# EUROPÄISCHE PATENTSCHRIFT

(11) **EP 3 634 532 B1**
(45) Veröffentlichungstag und Bekanntmachung des Hinweises auf die Patenterteilung: **18.12.2024**
(21) Anmeldenummer: 18731024.8
(22) Anmeldetag: 07.06.2018
(51) Int. Cl.: A61M 1/36, A61M 60/109, A61M 60/531, A61M 60/554

(54) **VORRICHTUNG ZUR EXTRAKORPORALEN BLUTBEHANDLUNG UND VORRICHTUNG ZUM SAMMELN VON BLUTGERINNSELN**
DEVICE FOR EXTRACORPOREAL BLOOD TREATMENT AND DEVICE FOR COLLECTING BLOOD CLOTS
DISPOSITIF POUR LE TRAITEMENT SANGUIN EXTRACORPOREL ET DISPOSITIF POUR LA COLLECTE DE CAILLOTS DE SANG

(30) Priorität: 10.06.2017 DE 102017005535
(43) Veröffentlichungstag der Anmeldung: 15.04.2020
(73) Patentinhaber: Fresenius Medical Care Deutschland GmbH, 61352 Bad Homburg v.d.H (DE)
(72) Erfinder: MAIERHOFER, Andreas, 97422 Schweinfurt (DE)
(74) Vertreter: Oppermann, Frank
(86) Internationale Anmeldenummer: PCT/EP2018/065057
(87) Internationale Veröffentlichungsnummer: WO 2018/224603

(56) Entgegenhaltungen:
- WO-A1-2010/121743
- WO-A1-2010/121750
- WO-A1-2012/022456
- WO-A1-2018/001996
- DE-C1- 19 528 907

## Beschreibung

Die Erfindung betrifft eine Vorrichtung zur extrakorporalen Blutbehandlung mit einer Blutbehandlungseinheit, die mindestens ein Kompartiment aufweist. Darüber hinaus betrifft die Erfindung eine Vorrichtung zum Sammeln von Blutgerinnseln für eine Blutleitung zum Zuführen oder Abführen von Blut in eine bzw. aus einer Blutbehandlungseinheit einer extrakorporalen Blutbehandlungsvorrichtung.

Bei Verfahren der chronischen Blutreinigungstherapie, wie Hämodialyse, Hämofiltration und Hämodiafiltration, wird Blut über einen extrakorporalen Blutkreislauf geleitet. Bei der Hamödialyse durchströmt das zu behandelnde Blut die Blutkammer eines durch eine semipermeable Membran in die Blutkammer und eine Dialysierflüssigkeitskammer unterteilten Dialysators, während in einem Dialysierflüssigkeitssystem Dialysierflüssigkeit die Dialysierflüssigkeitskammer des Dialysators durchströmt. Der extrakorporale Blutkreislauf weist eine arterielle Schlauchleitung auf, die zu der Blutkammer führt, und eine venöse Schlauchleitung auf, die von der Blutkammer abgeht. Die bekannten Blutbehandlungsvorrichtungen verfügen über eine Blutpumpe, die in der Regel stromauf der Blutkammer des Dialysators angeordnet ist, um einen ausreichenden Blutfluss im extrakorporalen Blutkreislauf sicherzustellen.

Als Zugang zum Blutgefäßsystem des Patienten wird häufig operativ eine arteriovenöse Fistel angelegt. Ebenso ist der Einsatz eines Implantats möglich. Für die Funktionsfähigkeit der Fistel ist deren Perfusion von Bedeutung. Sinkt der Fistelfluss unter einen kritischen Wert, dann steigt das Risiko einer Fistelthrombose mit dem möglichen Verlust des Gefäßzuganges, was in der Dialysebehandlung eine erhebliche Komplikation darstellt (W. Bay et al.: Color Doppler flow predicts PTFE graft failure, J. Am. Soc. Nephrol. 5: 407 (1994)). Ist der Fistelfluss während der Dialysebehandlung kleiner als der extrakorporale Blutfluss (Q_{B}), kommt es zur lokalen Fistelrezirkulation, wobei eine Fraktion des dialysierten und mit der venösen Blutleitung zur Fistel zurückgeführten Bluts über die arterielle Blutleitung dem Dialysator wieder zugeführt wird. Die Fistelrezirkulation (R_{A}) bewirkt eine bedeutende Verminderung der Dialyseeffizienz (F. Gotch: "Models to predict recirculation and its effect on treatment time in single-needle dialysis" First Intl. Symposium on Single-Needle Dialysis, Hrsg.: S. Rignoir. R. Vanholder und P. Invanovich, Cleveland, ISAO Press, 1984, Seite 305 ff.).

Aufgrund ihrer klinischen Bedeutung sind verschiedene Verfahren zur Bestimmung des Blutflusses im Gefäßzugang (Shuntfluss) oder der Rezirkulation bekannt. Viele Verfahren basieren auf der Messung einer physikalischen oder chemischen Kenngröße des Bluts, die im extrakorporalen Blutkreislauf verändert wird. Die physikalische oder chemische Kenngröße kann beispielsweise die Temperatur, Dichte oder Elektrolytzusammensetzung des Bluts sein. Die Kenngröße kann im extrakorporalen Blutkreislauf unmittelbar oder mittelbar durch eine Änderung einer physikalischen oder chemischen Kenngröße im Dialysierflüssigkeitssystem verändert werden.

Aus EDTNA-ERCA Journal 19, 6 (1993) ist ein als Thermodilution bezeichnetes Verfahren zur Shuntflussmessung bekannt. Bei dem bekannten Verfahren wird ein kurzzeitiger Temperaturabfall im Dialysierflüssigkeitssystem initiiert, der sich auf den venösen Zweig des extrakorporalen Kreislaufs überträgt und zu einem nachweisbaren Temperatursprung im arteriellen Zweig des extrakorporalen Kreislaufs dann führt, wenn eine Rezirkulation auftritt.

Die DE 195 28 907 C1 beschreibt ein Verfahren zum Bestimmen der kardiopulmonalen Rezirkulation, das auf zwei aufeinanderfolgenden Messungen der Rezirkulationsfraktion beruht, die vor und nach der Umkehr des Blutflusses im extrakorporalen Kreislauf durchgeführt werden. Eine Umkehrung des Blutflusses kann grundsätzlich durch Vertauschen der arteriellen und venösen Patientenanschlüsse erfolgen. Das manuelle Vertauschen der Patientenanschlüsse ist aber unter hygienischen und sicherheitstechnischen Gesichtspunkten nicht unproblematisch. Daher sind zahlreiche Vorrichtungen für Blutschlauchsysteme entwickelt worden, die eine automatische Umkehr der Flussrichtung durch eine kreuzweise Verschaltung der betreffenden Schlauchleitungsabschnitte des Schlauchleitungssystems bewirken. Dabei wird aber die Flussrichtung in dem Teil des extrakorporalen Kreislaufs, der den Dialysator einschließt, beibehalten. Daher können die in diesem Teil befindlichen Schutzsysteme sowohl vor als auch nach der Umkehr des Blutflusses verwendet werden.

Eine Ventileinrichtung für die automatische Umkehr der Flussrichtung ist beispielsweise aus der WO 2014/074231 A1 bekannt.

Die DE 195 28 907 C1 schlägt als eine Alternative zu den bekannten Ventileinrichtungen für die automatische Umkehr der Flussrichtung vor, einfach die Förderrichtung der Blutpumpe im extrakorporalen Blutkreislauf umzukehren. Allerdings ist von dieser Möglichkeit in der Praxis bisher kein Gebrauch gemacht worden, da sich der Betrieb einer extrakorporalen Blutbehandlungsvorrichtung mit einem Dialysator, der von Blut in unterschiedlichen Richtungen durchströmt wird, unter sicherheitstechnischen Gesichtspunkten als nicht unproblematisch erweist.

Aus der WO 2012/022456 A1 ist eine Vorrichtung zur Ermittlung oder Überwachung von Festkörpern oder Gasblasen in einem Fluid bekannt. Die WO 2012/022456 A1 weist auf die Problematik der Flussumkehr durch Änderung der Förderrichtung einer Blutpumpe hin. Die Lösung dieses Problems wird in der Überwachung des Blutstroms auf Festkörper oder Gasblasen gesehen. Für den Fall, dass Festkörper oder Gasblasen im Blut erkannt werden, sollen Schutzmaßnahmen vorgesehen werden, z. B. die Unterbrechung des Blutflusses.

Die WO 2010/121750 A1 beschreibt ein Verfahren zum Entfernen von Blut aus einem extrakorporalen Blutkreislauf nach Beendigen der Blutbehandlung. Die WO 2010/121750 A1 schlägt vor, in den arteriellen Abschnitt des extrakorporalen Blutkreislaufs einen Gerinnselfänger einzufügen.

Ein bekannter Gerinnselfänger ist beispielsweise in WO 2010/121743 A1 beschrieben.

Der Erfindung liegt die Aufgabe zugrunde, eine Messung eines hämodynamischen Parameters ohne die Verwendung der bekannten Ventileinrichtungen für die automatische Umkehr der Flussrichtung zu ermöglichen, die eine automatische Umkehr der Flussrichtung durch eine kreuzweise Verschaltung der betreffenden Schlauchleitungsabschnitte des Schlauchleitungssystems bewirken.

Die Lösung dieser Aufgabe erfolgt mit den Merkmalen der unabhängigen Patentansprüche. Die abhängigen Ansprüche betreffen bevorzugte Ausführungsformen der Erfindung.

Die erfindungsgemäße Vorrichtung zur extrakorporalen Blutbehandlung umfasst:
eine Blutbehandlungseinheit, die mindestens ein Kompartiment aufweist,
eine an einen Anschluss des Kompartiments angeschlossene erste Blutleitung, die einen ersten Patientenanschluss aufweist,
eine an einen Anschluss des Kompartiments angeschlossene zweite Blutleitung, die einen zweiten Patientenanschluss aufweist,
eine Blutpumpe zum Fördern von Blut,
eine Einrichtung zum Messen einer physikalischen oder chemischen Kenngröße,
eine mit der Blutpumpe und der Einrichtung zum Messen einer physikalischen oder chemischen Kenngröße verbundene Steuer- und Auswerteeinheit, die derart konfiguriert ist, dass
die Blutpumpe in einem ersten Betriebsmodus mit einem normalen Blutfluss betrieben wird, so dass Blut von dem ersten Patientenanschluss zu der Blutbehandlungseinheit und von der Blutbehandlungseinheit zu dem zweiten Patientenanschluss strömt,
die Blutpumpe in einem zweiten Betriebsmodus mit einem umgekehrten Blutfluss betrieben wird, so dass Blut von dem zweiten Patientenanschluss zu der Blutbehandlungseinheit und von der Blutbehandlungseinheit zu dem ersten Patientenanschluss strömt, und
aus der gemessenen physikalischen oder chemischen Kenngröße zumindest bei einem umgekehrten Blutfluss der hämodynamische Parameter bestimmt wird. Zur Vermeidung der Bildung von Blutgerinnseln im extrakorporalen Blutkreislauf wird häufig eine gerinnungshemmende Substanz, meist Heparin, entweder kontinuierlich während der Dialyse oder als initialer Bolus verabreicht. Die Zugabe erfolgt üblicherweise stromauf des Dialysators. Neben der systemischen Wirkung der gerinnungshemmenden Substanz im Patienten ist ein möglicher Wirkmechanismus auch die Anlagerung dieser Substanz an die Oberflächen des extrakorporalen Systems, insbesondere an das Lumen der Dialysatorfasern.

Der bei einer extrakorporalen Blutbehandlung verwendete Dialysator stellt wegen des Durchmessers der Hohlfasern von kleiner als 0,2 mm ein für Gerinnsel unüberwindliches Hindernis dar. Daher können stromauf des Dialysators sich bildende Gerinnsel nicht in den Bereich stromab des Dialysators und somit nicht in den Patienten gelangen. Allerdings können stromab des Dialysators befindliche Blutgerinnsel infundiert werden. Dies ist unabhängig von der Flussrichtung des Bluts durch den Dialysator.

Bei den bekannten Blutbehandlungsvorrichtungen werden sich im Blut auf dem Weg vom Dialysator zum Patienten bildende Gerinnsel im Allgemeinen mit einer Vorrichtung zum Fangen von Blutgerinnseln zurückgehalten, die stromab des Dialysators in der venösen Blutleitung des extrakorporalen Schlauchsystems vor der venösen Kanüle vorgesehen ist. Diese Vorrichtung wird auch als Blutgerinnselfänger bezeichnet.

In der Praxis hat sich gezeigt, dass bei einer Umkehr der Förderrichtung der Blutpumpe für eine Messung zur Bestimmung eines hämodynamischen Parameters das Risiko besteht, dass Blutgerinnsel in den Patienten gelangen, obwohl der Dialysator sich stromauf des Dialysators bildende Blutgerinnsel zurückhält. Es hat sich in der Praxis gezeigt, dass sich während eines Betriebs mit einem "normalen" Blutfluss am Eingang des Dialysators Blutgerinnsel ansammeln. Bei einer Umkehr des Blutflusses werden diese Blutgerinnsel dann in Richtung des Patienten gespült.

Die erfindungsgemäße Vorrichtung zur extrakorporalen Blutbehandlung sieht daher zumindest in der ersten Blutleitung eine Vorrichtung zum Sammeln von Blutgerinnseln vor. Während der Blutbehandlung mit einem "normalen" Blutfluss kann eine zweite Vorrichtung zum Fangen von Blutgerinnseln, die in der zweiten Blutleitung vorgesehen sein kann, stromab des Dialysators sich bildende Blutgerinnsel zurückhalten. Bei einem "umgekehrten" Blutfluss hält die Vorrichtung zum Sammeln von Blutgerinnseln in der ersten Blutleitung die Blutgerinnsel zurück, die sich zuvor am Eingang der Blutbehandlungseinheit angesammelt haben können. Folglich besteht nicht die Gefahr einer Infusion von Blutgerinnseln, wenn die Förderrichtung der Blutpumpe für die Bestimmung des hämodynamischen Parameters von einem "normalen" Blutfluss in einen "umgekehrten" Blutfluss umgeschaltet wird.

Für die Erfindung ist unerheblich, welche hämodynamischen Parameter auf der Grundlage einer oder mehreren Messungen bei einem umgekehrten Blutfluss bestimmt werden und wie die hämodynamischen Parameter bestimmt werden. Unerheblich ist auch, ob blutseitige oder dialysatseitige Größen gemessen werden. Anstelle der physikalischen oder chemischen Eigenschaft des Bluts in der Blutleitung kann auch eine mit der physikalischen oder chemischen Kenngröße in der Blutleitung korrelierende Kenngröße im Dialysat gemessen werden. Die Erfindung bezieht sich insofern auf sämtliche Verfahren, bei denen vor und nach der Umkehr der Flussrichtung oder nur bei einem umgekehrten Blutfluss eine chemische oder physikalische Kenngröße gemessen wird, um den hämodynamischen Parameter zu bestimmen.

Die Offenbarung bezieht sich beispielsweise auf sämtliche Verfahren, bei denen vor und nach der Umkehr der Flussrichtung eine chemische oder physikalische Kenngröße im Blut in einem Zweig des extrakorporalen Blutkreislaufs verändert und die auf die Veränderung der chemischen oder physikalischen Kenngröße in diesem Zweig zurückzuführende Änderung der chemischen oder physikalischen Kenngröße im Blut in einem anderen Zweig des extrakorporalen Blutkreislaufs oder im Dialysat erfasst wird.

Für die Bestimmung des hämodynamischen Parameters kann der Verlauf der beobachtbaren zeitlichen Veränderung der physikalischen oder chemischen Kenngröße im blutseitigen oder dialysatseitigen Leitungssystem gemessen werden. Ein konkreter Anwendungsfall ist die Bestimmung der Rezirkulation nach den bekannten Verfahren, die eine Umkehr der Flussrichtung voraussetzen. Der hämodynamische Parameter kann dabei die Rezirkulation in der Fistel oder der Shuntfluss sein.

Bei der Blutbehandlungseinheit kann es sich um eine beliebige Einheit zur Durchführung einer Hämodialyse, Hämofiltration, Hämodiafiltration, Apherese o.ä., handeln. Im Fall einer Hämodialyse, Hämofiltration, Hämodiafiltration weist die Blutbehandlungseinheit (Dialysator) ein erstes und ein zweites Kompartiment auf, die durch eine semipermeable Membran voneinander getrennt sind. Wenn die Blutbehandlung hingegen nicht auf einem diffusiven oder konvektiven Stoffaustausch, beispielsweise auf einer Adsorption an funktionalisierten Oberflächen beruht, so ist ein zweites Kompartiment nicht erforderlich.

Nach der Umkehr der Flussrichtung des Bluts können mit einer Vorrichtung zum Fangen von Blutgerinnseln in der ersten Blutleitung Blutgerinnsel, die sich vor der Umkehrung der Flussrichtung des Blutes im Blut stromauf des Kompartiments der Blutbehandlungseinheit gebildet haben, zurückgehalten werden.

Darüber hinaus hat sich in der Praxis gezeigt, dass die erste Vorrichtung zum Fangen von Blutgerinnseln in der ersten Blutleitung bei einem "normalen" Blutfluss zu einer Erhöhung des Flusswiderstandes führt. Dies ist insofern nachteilig, als für den "normalen" Blutfluss die erste Vorrichtung zum Fangen von Blutgerinnseln eigentlich nicht erforderlich ist, da die Blutgerinnsel von der Blutbehandlungseinheit bereits wirkungsvoll zurückgehalten werden.

Eine Ausführungsform sieht daher eine flussrichtungsabhängige Vorrichtung zum Fangen von Blutgerinnseln vor. Zumindest eine der beiden Vorrichtungen zum Fangen von Blutgerinnseln ist bei der bevorzugten Ausführungsform flussrichtungsabhängig. Insbesondere die bei einem "normalen" Blutfluss stromauf des Dialysators vorgesehene erste Vorrichtung zum Fangen von Blutgerinnseln ist nur dann zumindest voll wirksam, wenn die Flussrichtung umgekehrt ist. Ansonsten wird die Strömung des Bluts durch die Vorrichtung zum Fangen von Blutgerinnseln zumindest nicht wesentlich beeinträchtigt. Daher wird nicht nur eine Erhöhung des Strömungswiderstands des Blutes vermieden, sondern auch die Gefahr einer Schädigung des Bluts oder der Schaffung von Stagnationsstellen verringert, die wiederum zur Bildung von Blutgerinnseln führen können.

Bei der erfindungsgemäßen Vorrichtung zur extrakorporalen Blutbehandlung weist die Vorrichtung zum Sammeln von Blutgerinnseln einen Gehäusekörper mit einem ersten Anschluss und einem zweiten Anschluss und einen im Strömungsweg des Bluts zwischen den beiden Anschlüssen angeordneten Siebkörper zum Fangen der Blutgerinnsel auf. Der Siebkörper kann unterschiedlich ausgebildet sein, solange Blutgerinnsel wirkungsvoll zurückgehalten werden. Derartige Filtersiebe gehören zum Stand der Technik. Die Vorrichtung zum Sammeln von Blutgerinnseln ist derart ausgebildet, dass sich in dem Gehäusekörper ein den Siebkörper umgehender Strömungsweg ausbildet, wenn Blut nicht in der "normalen"

Strömungsrichtung an dem ersten Anschluss zufließt und an dem zweiten Anschluss abfließt, sondern in der "umgekehrten" Strömungsrichtung an dem zweiten Anschluss zufließt und an dem ersten Anschluss abfließt. Da zumindest ein Teil des Blutstroms den Siebkörper umgeht, ist der Strömungswiderstand verringert. Wenn Blut in der "normalen" Strömungsrichtung an dem ersten Anschluss zufließt und an dem zweiten Anschluss abfließt, bildet sich hingegen in dem Gehäusekörper ein den Siebkörper umgehender Strömungsweg nicht aus. In dieser Strömungsrichtung kann die Vorrichtung die Blutgerinnsel wirkungsvoll zurückhalten.

Eine weitere Ausführungsform sieht vor, dass der Siebkörper als Ventilkörper ausgebildet ist und in dem Gehäusekörper ein Ventilsitz für den Siebkörper ausgebildet ist. Der Siebkörper ist in dem Gehäusekörper derart bewegbar angeordnet, dass dieser auf dem Ventilsitz aufsitzt, wenn Blut in der "normalen" Flussrichtung an dem ersten Anschluss zufließt und an dem zweiten Anschluss abfließt, und von dem Ventilsitz abgehoben ist, wenn Blut an dem zweiten Anschluss zufließt und an dem ersten Anschluss abfließt, so dass sich in dem Gehäusekörper ein den Siebkörper umgehender Strömungsweg ausbildet. Die Bewegung des Ventilkörpers kann allein durch den Staudruck erfolgen, wenn das Blut in Richtung des Ventilkörpers strömt.

Ein Aufsitzen des Siebkörpers auf den Ventilsitz nur in einer Strömungsrichtung kann durch eine besondere Form des Gehäuses und/oder die Beschränkung des Bereichs erreicht werden, in dem sich der Siebkörper in dem Gehäuse bewegen kann.

Bei einer Ausführungsform ist der Siebkörper im Strömungsweg des Bluts zwischen dem ersten und zweiten Anschluss bewegbar angeordnet ist, wobei die Bewegung des Siebkörpers in dem Gehäusekörper auf einen Bereich zwischen einer ersten und einer zweiten Endposition beschränkt ist. Die Beschränkung des Bewegungsbereichs des Siebkörpers im Strömungsweg des Bluts zwischen dem ersten und zweiten Anschluss kann mit mindestens einem langgestreckten, flexiblen Fixierungselement erfolgen, dessen eines Ende mit dem Siebkörper und dessen anderes Ende mit dem Gehäusekörper verbunden ist. Bei einer bevorzugten Ausführungsform sind mehrere langgestreckte, flexible Fixierungselemente vorgesehen, die um den Umfang des Siebkörpers angeordnet sind. Dadurch kann der Siebkörper von allen Seiten in der Mitte des Gehäusekörpers zentral ausreichend fixiert werden, wobei das Blut zwischen den Fixierungselementen durch den Gehäusekörper ungehindert strömen kann, wenn der Siebkörper nicht auf dem Ventilsitz aufsitzt.

Eine weitere Ausführungsform sieht vor, dass der Gehäusekörper einen kreisförmigen Querschnitt aufweist und der Siebkörper ein Halteteil mit einem kreisförmigen Querschnitt aufweist, in das ein Sieb eingesetzt ist. Das Sieb kann als flacher scheibenförmiger Körper oder in der Form eines Fingerhuts ausgebildet ist. Die fingerhutförmige Ausbildung des Siebs hat den Vorteil einer vergrößerten Oberfläche. Dadurch kann sich das Sieb nicht so schnell zusetzten.

Der erste und zweite Anschluss der Vorrichtung zum Fangen von Blutgerinnseln kann beispielsweise als ein Anschlussteil eines Luer oder Luer/Lock-Anschlusses ausgebildet sein, um die Vorrichtung an die entsprechenden Schlauchleitungsabschnitte des Blutschlauchsystems bzw. der Patientenanschlüsse (Punktionskanülen) anschließen zu können. In diesem Fall kann die Vorrichtung zum Fangen von Blutgerinnseln zusammen mit dem Blutschlauchsystem oder ohne das Blutschlauchsystem bereitgestellt werden. Die Vorrichtung zum Fangen von Blutgerinnseln kann Bestandteil der Schlauchleitung des Blutschlauchsystems oder der Punktionskanüle sein. Beispielsweise können die Anschlüsse mit der Schlauchleitung oder der Kanüle verklebt oder verschweißt sein.

Bei einer Ausführungsform ist in der ersten Blutleitung eine erste Vorrichtung zum Sammeln von Blutgerinnseln und in der zweiten Blutleitung eine zweite Vorrichtung zum Sammeln von Blutgerinnseln vorgesehen.

Wenn der mit dem ersten Patientenanschluss verbundene Abschnitt der ersten Blutleitung an dem zweiten Anschluss des Gehäusekörpers der ersten Vorrichtung zum Sammeln von Blutgerinnseln angeschlossen ist und der mit dem Anschluss der Blutbehandlungseinheit verbundene Abschnitt der ersten Blutleitung an dem ersten Anschluss des Gehäusekörpers der ersten Vorrichtung zum Sammeln von Blutgerinnseln angeschlossen ist, kann das Blut in der "normalen" Flussrichtung an dem Filtersieb vorbei durch die erste Vorrichtung zum Fangen von Blutgerinnseln strömen. In der "umgekehrten" Flussrichtung hält die erste Vorrichtung hingegen Blutgerinnsel zurück.

Wenn der mit der Blutbehandlungseinheit verbundene Abschnitt der zweiten Blutleitung an dem ersten Anschluss des Gehäusekörpers der zweiten Vorrichtung zum Sammeln von Blutgerinnseln angeschlossen ist und der mit dem zweiten Patientenanschluss verbundene Abschnitt der zweiten Blutleitung an dem zweiten Anschluss des Gehäusekörpers der zweiten Vorrichtung zum Sammeln von Blutgerinnseln angeschlossen ist, hält die zweite Vorrichtung in der "normalen" Flussrichtung Blutgerinnsel zurück. In der "umgekehrten" Flussrichtung hält die zweite Vorrichtung hingegen Blutgerinnsel nicht zurück.

Insbesondere wenn die Flussumkehr erst zu einem späten Zeitpunkt der Blutbehandlung erfolgt, besteht die Gefahr, dass sich stromauf der Blutbehandlungseinheit eine relativ große Menge an Blutgerinnseln angesammelt haben, die nach einer Flussumkehr die erste Vorrichtung zum Fangen von Blutgerinnseln verstopfen könnten. Eine weitere Ausführungsform sieht daher einen erster Drucksensor zum Messen des Drucks in der ersten Blutleitung vor, der mit der Steuer- und Auswerteeinheit verbunden ist, so dass die Steuer- und Auswerteeinheit die Messwerte des ersten Drucksensors empfängt. Die Steuer- und Auswerteeinheit ist derart konfiguriert, dass der mit dem ersten Drucksensor in dem zweiten Betriebsmodus gemessene Druck mit einem vorgegebenen Grenzwert verglichen wird. Wenn der mit dem ersten Drucksensor gemessene Druck den vorgegebenen Grenzwert überschreitet, wird ein das Verstopfen der Vorrichtung signalisierendes Signal erzeugt wird, das ein weiteres Ereignis auslösen kann. Der Grenzwert kann ein absoluter Grenzwert sein, der in einem Speicher der Steuer- und Auswerteinheit gespeichert ist, oder ein vom Blutfluss abhängiger Grenzwert sein, der von der Steuer- und Auswerteinheit unter Berücksichtigung der vorgegebenen Blutflussrate berechnet wird.

Die Steuer- und Auswerteeinheit ist bei einer weiteren Ausführungsform derart konfiguriert, dass die Blutpumpe von dem "umgekehrten" Blutfluss auf den "normalen" Blutfluss umgeschaltet wird, wenn das Signal erzeugt wird. Bei einer Detektion einer Filterverstopfung kann die Flussumkehr auch für den Rest der Blutbehandlung von der Steuer- und Auswerteeinheit verhindert werden. Zudem kann eine Benutzersteuerung derart ausgelegt werden, dass die Sperre der Flussumkehr durch eine Bestätigung wieder aufgehoben werden kann, dass sich eventuelle Gerinnsel inzwischen durch Gabe von Antikoagulantia wieder aufgelöst haben. Mit der Steuer- und Auswerteeinheit kann auch eine Alarmeinheit und/oder Anzeigeeinheit verbunden sein, so dass die Alarmeinheit und/oder Anzeigeeinheit das Signal empfängt.

Eine alternative Ausführungsform sieht vor, dass ein erster Drucksensor zum Messen des Drucks in der ersten Blutleitung und ein zweiter Drucksensor zum Messen des Drucks in der zweiten Blutleitung mit der Steuer- und Auswerteeinheit verbunden sind, so dass die Steuer- und Auswerteeinheit die Messwerte des ersten und zweiten Drucksensors empfängt. Die Steuer- und Auswerteeinheit ist bei dieser Ausführungsform derart konfiguriert, dass der mit dem ersten Drucksensor in dem zweiten Betriebsmodus gemessene Druck mit dem Druck verglichen wird, der mit dem zweiten Drucksensor in dem ersten Betriebsmodus gemessen wird. Wenn der mit dem ersten Drucksensor gemessene Druck den mit dem zweiten Drucksensor gemessenen Druck um einen vorgegebenen Betrag oder Faktor überschreitet, wird ein Signal erzeugt, das eine Verstopfung signalisiert. Der Druck wird mit dem ersten und zweiten Drucksensor vorzugsweise bei der gleichen Blutflussrate gemessen, so dass der Blutfluss keinen Einfluss auf die Überwachung der Vorrichtung zum Fangen von Blutgerinnseln ausüben kann.

Im Folgenden werden verschiedene Ausführungsbeispiele der Erfindung unter Bezugnahme auf die Zeichnungen näher erläutert.

Es zeigen:
- Fig. 1: Ein Ausführungsbeispiel der erfindungsgemäßen Vorrichtung in stark vereinfachter schematischer Darstellung,
- Fig. 2A: ein erstes Ausführungsbeispiel einer Vorrichtung zum Fangen von Blutgerinnseln in Durchflussrichtung,
- Fig. 2B: ein erstes Ausführungsbeispiel einer Vorrichtung zum Fangen von Blutgerinnseln entgegen der Durchflussrichtung,
- Fig. 2C: den Siebkörper der Vorrichtung zum Fangen von Blutgerinnseln in der Draufsicht,
- Fig. 3A: ein zweites Ausführungsbeispiel einer Vorrichtung zum Fangen von Blutgerinnseln in Durchflussrichtung und
- Fig. 3B: ein zweites Ausführungsbeispiel einer Vorrichtung zum Fangen von Blutgerinnseln entgegen der Durchflussrichtung.

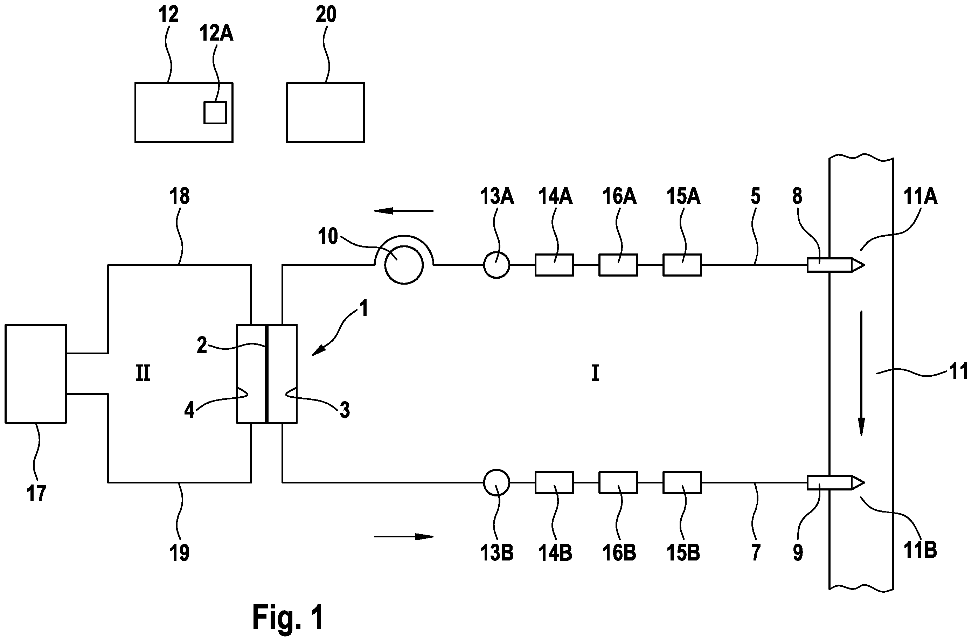

Fig. 1 zeigt in stark vereinfachter schematischer Darstellung die für die Erfindung wesentlichen Komponenten der Blutbehandlungsvorrichtung, die bei dem vorliegenden Ausführungsbeispiel eine Hämodialysevorrichtung ist. Die Blutbehandlungsvorrichtung weist eine Blutbehandlungseinheit 1 auf, die ein Dialysator sein kann, der durch eine semipermeable Membran 2 in ein erstes Kompartiment 3 (Blutkammer) und ein zweites Kompartiment 4 (Dialysatkammer) unterteilt ist. Der extrakorporale Blutkreislauf I umfasst eine erste Blutleitung 5, die an einen ersten Anschluss des ersten Kompartiments 3 angeschlossen ist, das erste Kompartiment 3 und eine zweite Blutleitung 7, die an einen zweiten Anschluss des ersten Kompartiments 3 angeschlossen ist. Die erste und zweite Blutleitung 5, 7 sind Schlauchleitungen eines zur einmaligen Verwendung bestimmten Blutschlauchsystems (Disposable). An dem Ende der ersten Blutleitung 5 befindet sich ein erster Patientenanschluss 8 (Punktionskanüle) und an dem Ende der zweiten Blutleitung 7 befindet sich ein zweiter Patientenanschluss 9 (Punktionskanüle). Das Blut wird mit einer Blutpumpe 10 gefördert, die in der ersten Blutleitung 5 vorgesehen ist.

Zur Durchführung der Blutbehandlung wird die erste Punktionskanüle 8 an einem stromaufwärtigen Teil 1lA des Gefäßzugangs 11 angeschlossen, während die zweite Punktionskanüle 9 an einem stromabwärtigen Teil 11B des Gefäßzugangs 11 angeschlossen wird, so dass das Blut des Patienten durch die erste Blutleitung 5, das erste Kompartiment 3 der Blutbehandlungseinheit 3 und die zweite Blutleitung 7 strömt. Dieser Fall wird als "normaler" Blutfluss bezeichnet. Die Blutpumpe 10 fördert dann in der "normalen" Förderrichtung. Der stromaufwärtige Teil 11A des Gefäßzugangs 11 stellt somit den arteriellen Teil der Fistel, die erste Blutleitung 5 die arterielle Blutleitung, die zweite Blutleitung 7 die venöse Blutleitung und der stromabwärtige Teil 11B der Fistel den venösen Teil der Fistel dar.

Für die Messung des hämodynamischen Parameters ist eine Umkehr der Flussrichtung erforderlich, so dass das Blut von dem stromabwärtigen Teil 11B des Gefäßzugangs 11 durch die zweite Blutleitung 7, das erste Kompartiment 3 der Blutbehandlungseinheit 1, die erste Blutleitung 5 zu dem stromaufwärtigen Teil 11A des Gefäßzugangs 11 strömt. Dieser Fall wird als "umgekehrter" Blutfluss bezeichnet. Die Blutpumpe 10 fördert dann in der "umgekehrten" Förderrichtung.

Die Blutbehandlungsvorrichtung verfügt über eine zentrale Steuer- und Auswerteeinheit 12 zur Steuerung sämtlicher Komponenten der Vorrichtung. Die Steuer- und Auswerteeinheit 12 kann beispielsweise einen allgemeinen Prozessor, einen digitalen Signalprozessor (DSP) zur kontinuierlichen Bearbeitung digitaler Signale, einen Mikroprozessor, eine anwendungsspezifische integrierte Schaltung (ASIC), einen aus Logikelementen bestehenden integrierten Schaltkreis (FPGA) oder andere integrierte Schaltkreise (IC) oder Hardware-Komponenten aufweisen, um die einzelnen Verfahrensschritte zur Steuerung der Blutbehandlungsvorrichtung auszuführen. Auf den Hardware-Komponenten kann zur Durchführung der Verfahrensschritte ein Datenverarbeitungsprogramm (Software) laufen. Es ist auch eine Mehrzahl oder Kombination der verschiedenen Komponenten möglich.

Die Steuer- und Auswerteeinheit 12 ist derart konfiguriert, dass die einzelnen Komponenten der Blutbehandlungsvorrichtung für die Durchführung der Blutbehandlung angesteuert und die Messungen für die Bestimmung eines hämodynamischen Parameters der Blutbehandlung durchgeführt werden.

Die Blutbehandlungsvorrichtung weist in der ersten Blutleitung 5 zwischen der Blutpumpe 10 und dem ersten Patientenanschluss 8 einen ersten Drucksensor 13A zum Messen des Drucks und in der zweiten Blutleitung 7 zwischen dem ersten Kompartiment 3 und dem zweiten Patientenanschluss 9 einen zweiten Drucksensor 13B zum Messen des Drucks auf.

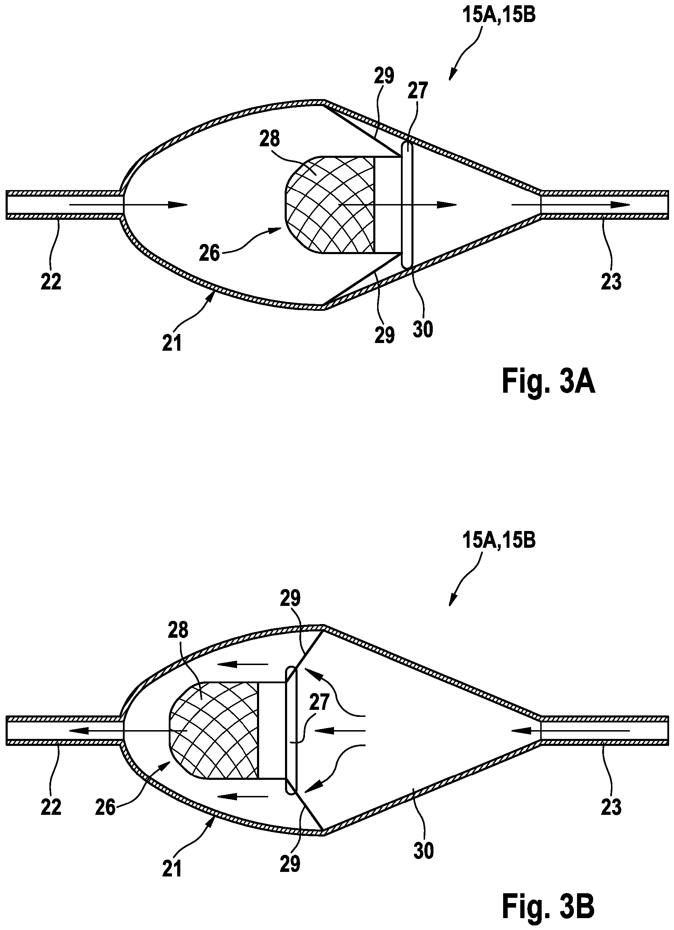

Die Messungen für die Bestimmung eines hämodynamischen Parameters der Blutbehandlung können im extrakorporalen Blutkreislauf I durchgeführt werden. Zum Messen einer physikalischen oder chemischen Eigenschaft des Bluts in der ersten Blutleitung ist eine erste Messeinrichtung 14A und zum Messen einer physikalischen oder chemischen Eigenschaft des Bluts in der zweiten Blutleitung ist eine zweite Messeinrichtung 14B vorgesehen. Die Drucksensoren 13A, 13B und die Einrichtungen 14A, 14B zum Messen einer physikalischen oder chemischen Eigenschaft des Bluts sind über nicht dargestellte Datenleitungen mit der Steuer- und Auswerteeinheit 12 verbunden. In der ersten Blutleitung 5 ist zwischen dem ersten Patientenanschluss 8 und der ersten Einrichtung 14A zum Messen einer physikalischen oder chemischen Eigenschaft eine erste Vorrichtung 15A zum Fangen von Blutgerinnseln und in der zweiten Blutleitung 7 ist zwischen der zweiten Einrichtung 14B zum Messen einer physikalischen oder chemischen Eigenschaft und dem zweiten Patientenanschluss 9 eine zweite Vorrichtung 15B zum Fangen von Blutgerinnseln vorgesehen. Die Vorrichtungen 15A, 15B zum Fangen von Blutgerinnseln werden unter Bezugnahme auf die Figuren 3A, 3B und 4A und 4B nachfolgend noch im Einzelnen beschrieben. Die Blutgerinnselfänger 15A, 15B können aber auch in anderen Abschnitten der ersten und zweiten Blutleitung 5, 7 vorgesehen sein. Darüber hinaus weist die Blutbehandlungsvorrichtung weitere nur andeutungsweise dargestellte Überwachungs- und Sicherheitseinrichtungen 16A, 16B auf, die sowohl in der "normalen" als auch "umgekehrten" Flussrichtung funktionsfähig sind. Zu diesen Überwachungs- und Sicherheitseinrichtungen zählen 16A, 16B eine Luftabscheidekammer, ein Luftdetektor und ein Absperrorgan in der ersten und zweiten Blutleitung. Die Luftabscheidekammern können Ein- und Auslassleitungen haben, die unterhalb des Flüssigkeitsspiegels angeordnet sind. Zur Erkennung von Luft können Ultraschallsensoren verwendet werden.

Das Dialysierflüssigkeitssystem II umfasst eine Einrichtung 17 zum Aufbereiten von Dialysierflüssigkeit mit einer vorgegebenen Temperatur. Die frische Dialysierflüssigkeit strömt über eine Dialysierflüssigkeitszuführleitung 18 in das zweite Kompartiment 4 und die gebrauchte Dialysierflüssigkeit aus dem zweiten Kompartiment 4 der Blutbehandlungseinheit 1 über eine Dialysierflüssigkeitsabführleitung 19 zu der Einrichtung 17 zum Aufbereiten von Dialysierflüssigkeit. Die Einrichtung 17 zum Aufbereiten von Dialysierflüssigkeit ist über eine nicht dargestellte Datenleitung mit der Steuer- und Auswerteeinheit 12 verbunden, so dass die Steuer- und Auswerteeinheit 12 die Temperatur und Zusammensetzung der Dialysierflüssigkeit steuern kann.

Die Messungen für die Bestimmung eines hämodynamischen Parameters der Blutbehandlung können anstelle im extrakorporalen Blutkreislauf I auch im Dialysierflüssigkeitssystem II durchgeführt werden. Diese Verfahren gehören zum Stand der Technik.. So kann z.B. durch Messung der Clearance bei normalem und bei umgekehrtem Blutfluss der Fistelfluss bestimmt werden.

Für den Fall einer Hämofiltration braucht eine Einrichtung zum Aufbereiten von Dialysierflüssigkeit und eine zu dem zweiten Kompartiment der Blutbehandlungseinheit führende Dialysierflüssigkeitszuführleitung nicht vorhanden zu sein. Ultrafiltrat kann aus dem zweiten Kompartiment der Blutbehandlungseinheit über die Dialysierflüssigkeitsabführleitung abgezogen werden. Für eine Blutbehandlung, bei der ein diffusiver oder konvektiver Austausch nicht stattfindet, braucht auch ein zweites Kompartiment und eine Dialysierflüssigkeitszu- und abführleitung nicht vorhanden zu sein. Für die Erfindung ist nur entscheidend, dass die Bestimmung des hämodynamischen Parameters eine Flussumkehr erfordert. Für die Erfindung ist nicht entscheidend, ob für die Bestimmung des hämodynamischen Parameters im Blut eine physikalische oder chemische Kenngröße verändert wird. Die Messungen der physikalischen oder chemischen Kenngröße können auf der Blutseite oder Dialysatseite erfolgen.

Bei dem vorliegenden Ausführungsbeispiel fungiert die Einrichtung 17 zum Aufbereiten von Dialysierflüssigkeit auch als Einrichtung zur Veränderung einer physikalischen oder chemischen Eigenschaft des Bluts. Hierfür verändert die Einrichtung 17 zum Aufbereiten von Dialysierflüssigkeit kurzzeitig die Temperatur oder Zusammensetzung der Dialysierflüssigkeit im Dialysierflüssigkeitssystem II, was eine Änderung einer physikalischen oder chemischen Kenngröße im extrakorporalen Blutkreislauf I zur Folge hat. Die Änderung der physikalischen oder chemischen Eigenschaft im Blut, beispielsweise der Temperatur des Bluts (Temperarturbolus) wird mit der ersten oder zweiten Messeinrichtung 14A, 14B in der ersten und zweiten Blutleitung 5, 7 gemessen. Alternativ kann z.B. aus dem Verlauf der Leitfähigkeit der Dialysierflüssigkeit, die mit nicht gezeigten Leitfähigkeitszellen in der Dialysierflüssigkeitszuführleitung 18 und der Dialysierflüssigkeitsabführleitung 19 gemessen werden kann, der hämodynamische Parameter, insbesondere der Shuntfluss, bestimmt werden. Alternativ kann die Änderung der physikalischen oder chemischen Eigenschaft des Bluts auch durch externe Elemente wie am Blutschlauchsystem angebrachte Peltierelemente oder die Infusion einer physiologischen Lösung über eine in der ersten oder zweiten Blutleitung gelegene Zugabestelle erfolgen.

Die Laufrichtung und Förderrate der Blutpumpe 10 kann mit der Steuer- und Auswerteeinheit 12 geregelt werden. Hierzu können die Druckwerte der Drucksensoren 13A, 13B verwendet werden, um in bekannter Weise aus Umdrehungszahl der Pumpe und Unterdruck auf der jeweiligen Saugseite den effektiven Blutfluss zu bestimmen und zu korrigieren.

Der zeitliche Verlauf der physikalischen oder chemischen Eigenschaft des entnommenen Bluts und des zurückgegebenen Bluts wird mit den am Blutschlauchsystem vorgesehenen Messeinrichtungen 14A, 14B gemessen und an die Steuer- und Auswerteeinheit 12 übermittelt. Bei den Messeinrichtungen 14A, 14B kann es sich beispielsweise um Temperatursensoren, optische oder spektroskopische Sensoren oder Dichtesensoren, beispielsweise Ultraschallsensoren, handeln. Ebenso können Leitfähigkeitsmesszellen verwendet werden, um mit den betreffenden physikalischen oder chemischen Eigenschaften des Bluts korrelierende Größen zu bestimmen. Anstelle der blutseitigen physikalischen oder chemischen Kenngrößen können auch dialysatseitige physikalische oder chemische Kenngrößen gemessen werden, die mit den blutseitigen physikalischen oder chemischen Kenngrößen korrelieren. Hierzu können Messwertaufnehmer zum Messen der Kenngrößen im Dialysierflüssigkeitssystem II, beispielsweise in einer oder beiden Dialysierflüssigkeitsleitungen 18 und 19 vorgesehen sein.

Bei dem vorliegenden Ausführungsbeispiel wird als hämodynamischer Parameter der Fluss im Gefäßzugang (Shuntfluss) bestimmt. Die Bestimmung des Shuntflusses kann mit dem Verfahren erfolgen, das in der DE 195 28 907 A1 im Einzelnen beschrieben ist.

Der Ablauf der Shuntflussmessung wird durch die Steuer- und Auswerteeinheit 12 gesteuert. Zunächst wird die Blutpumpe 10 in der "normalen" Förderrichtung betrieben. Die Temperatur oder Zusammensetzung der Dialysierflüssigkeit wird bei "normalem" Blutfluss kurzzeitig verändert. Der Temperaturbolus oder Leitfähigkeitssprung überträgt sich infolge des Wärmeaustausches am Dialysator auf den extrakorporalen Blutkreislauf I. Die Messeinrichtungen 14A, 14B in der ersten und zweiten Blutleitung 5, 7 erfassen die Änderungen der physikalischen oder chemischen Kenngröße in der ersten und zweiten Blutleitung. Die Messwerte werden in einer Speichereinheit 12A der Steuer- und Auswerteeinheit 12 gespeichert. Daraufhin wird die Blutpumpe 10 in der "umgekehrten" Förderrichtung betrieben. Die Temperatur oder Zusammensetzung der Dialysierflüssigkeit wird bei "umgekehrtem" Blutfluss kurzzeitig verändert und die Temperatur oder Zusammensetzung der Dialysierflüssigkeit in der ersten und zweiten Blutleitung gemessen. Die Messwerte werden wieder in der Speichereinheit 12A der Steuer- und Auswerteeinheit 12 gespeichert. Die Steuer- und Auswerteeinheit 12 berechnet nunmehr auf der Grundlage der Messwerte der Messeinrichtungen unter Anwendung einer geeigneten Ablaufsteuerung und entsprechender Auswerteverfahren den Shuntfluss und zeigt das Ergebnis auf einer Anzeigeeinheit 20 an oder gibt das Ergebnis über eine andere Art der Kommunikation, beispielsweise über ein Netzwerk, aus. Auf der Anzeigeeinheit 20 können auch eventuelle Fehlermeldungen oder Handlungsanweisungen dem Anwender übermittelt werden. Anstelle einer Anzeigeeinheit kann auch eine Alarmeinheit vorgesehen sein, auf der ein akustischer oder optischer oder taktiler Alarm gegeben wird, beispielsweise bei einer Verstopfung des Filters. Das Auswertverfahren zur Berechnung des Shuntflusses aus den Messwerten ist in der DE 195 28 907 A1 beschrieben, auf die ausdrücklich Bezug genommen wird.

Die Steuer- und Auswerteeinheit 12 stellt während der Umkehr der Flussrichtung sicher, dass die für Dialysebehandlungen vorgesehenen Schutzeinrichtungen 16A, 16B an die Umkehr der Blutflussrichtung angepasst werden. Hierzu gehört insbesondere die arterielle und venöse Drucküberwachung, deren Bedeutung bei der Flussumkehr vertauscht wird. Während auf der Saugseite der Blutpumpe ("arteriell") ein Unterdruck anliegt, ist der Druck auf der Rückgabeseite ("venös") positiv. Zugleich dient der venöse Druck zur Detektion eventueller Nadeldiskonnektionen. Die Steuer- und Auswerteeinheit ist daher derart konfiguriert, dass das vorgegebene arterielle und venöse Druckgrenzwertfenster bei einer Flussumkehr vertauscht werden. Mit den Algorithmen zur Detektion einer Nadeldiskonnektion, die auf einer dynamischen Signalanalyse beruhen und/oder das absolute Unterschreiten einer unteren Druckschwelle auf der Rückgabeseite überwachen, werden im Normalbetrieb anstelle der Messwerte des venösen Sensors nun die Messwerte des arteriellen Sensor ausgewertet. Zudem erfolgt nach der Flussumkehr eine Überwachung auf Luftinfusion auf der im Normalbetrieb arteriellen Seite.

Bei dem vorliegenden Ausführungsbeispiel erfolgt die Messung zunächst bei einem "normalen" Blutfluss. Es ist aber auch möglich, dass die Messung zunächst mit einem "umgekehrten" Blutfluss erfolgt.

Nachfolgend wird der Aufbau der in der ersten und zweiten Blutleitung vorgesehenen Vorrichtungen zum Fangen der Blutgerinnsel im Einzelnen beschrieben.

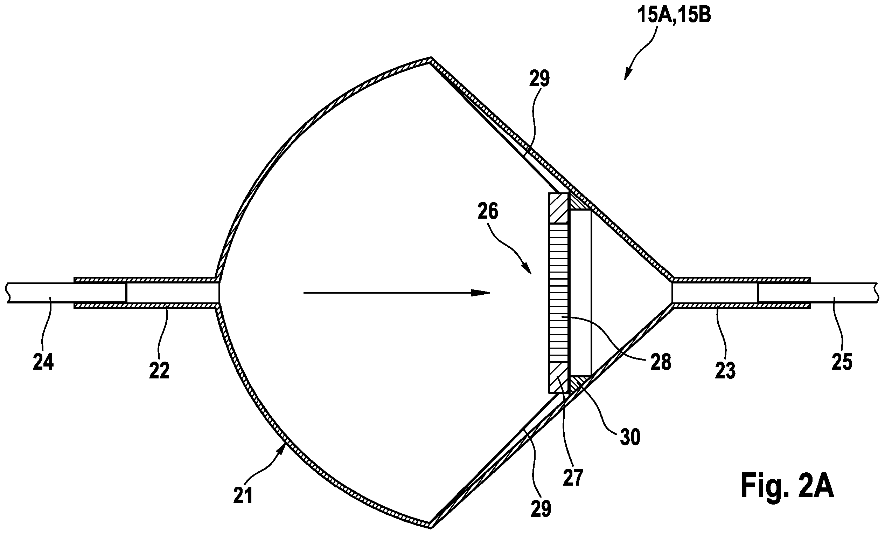

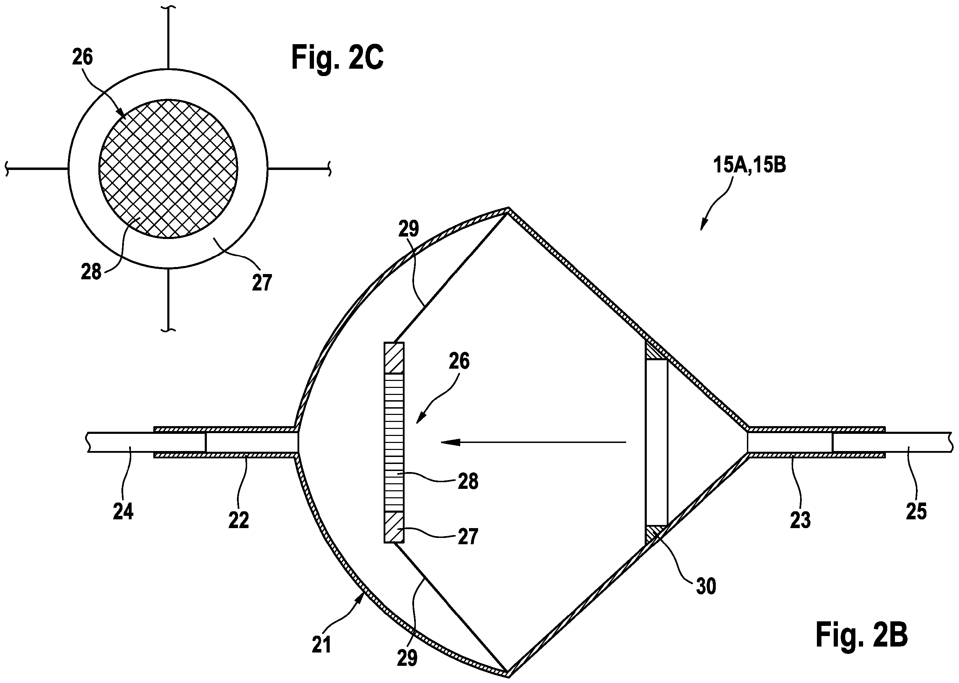

Die Figuren 2A und 2B zeigen in vereinfachter schematischer Darstellung einen Schnitt durch ein erstes Ausführungsbeispiel des Blutgerinnselfängers 15A oder 15B, der nur in einer Flussrichtung wirksam ist. In der entgegengesetzten Flussrichtung ist der Blutgerinnselfänger nicht wirksam, so dass der Flusswiderstand geringer ist. Die Flussrichtung ist in den Figuren 2A und 2B mit einem Pfeil gekennzeichnet.

Der Blutgerinnselfänger weist einen vorzugsweise rotationssymmetrischen Gehäusekörper 21 auf, der einen ersten Anschluss 22 und einen zweiten Anschluss 23 hat. Die Anschlüsse können Luer oder Luer/Lock-Anschlüsse sein oder Anschlussstutzen, die mit den Schlauchleitungsabschnitten 24, 25 des Blutschlauchsystems verschweißt oder verklebt sind. In dem Gehäusekörper 21 befindet sich ein flacher kreisförmiger Siebkörper 26, der ein ringförmiges Halteteil 27 aufweist, in das ein kreisförmiges Sieb 28 eingesetzt ist (Fig. 2C). Die Maschenweite des Siebs 28 ist derart bemessen, dass Blutgerinnsel zurückgehalten werden. Das ringförmige Halteteil 27 hat einen kleineren Außendurchmesser als der Innendurchmesser des Gehäusekörpers 21, so dass das Halteteil 27 bzw. der Siebkörper 26 seitlich von Blut umströmt werden kann. Der Siebkörper 26 ist in dem Strömungsweg des Bluts mittig in dem Gehäusekörpers 21 bewegbar angeordnet. Das Halteteil 27 ist hierzu in dem Gehäusekörper 21 an umfangsmäßig verteilt angeordneten flexiblen Fixierungselementen 29, insbesondere Fäden, aufgehängt. Die einen Enden der Fäden 29 sind mit dem Halteteil 27 verbunden und die anderen Enden der Fäden sind an der Wandung des Gehäusekörpers 21 befestigt. Die Länge der Fäden 29 ist derart bemessen, dass sich der Siebkörper 26 zwischen der in Fig. 2A gezeigten ersten Position und der in Fig. 2B gezeigten zweiten Position frei in der Mitte des Gehäusekörpers 21 bewegen kann.

An der dem zweiten Anschluss 23 zugewandten Seite ist in dem Gehäusekörper 21 ein Ventilsitz 30 ausgebildet, auf dem das Halteteil 27 des Siebkörpers 26 in der zweiten Position abdichtend aufsitzt (Fig. 2A). Auf der dem ersten Anschluss 22 zugewandten Seite ist in dem Gehäusekörper 21 ein Ventilsitz nicht ausgebildet, so dass der Siebkörper 26 seitlich von Blut umströmt werden kann (Fig. 2B). Auf der Seite des Ventilsitzes 30 hat der Gehäusekörper 21 eine Trichterform und auf der gegenüberliegenden Seite eine Kalottenform.

Wenn das Blut an dem ersten Anschluss 22 in den Gehäusekörper 21 strömt, bewegt sich der Siebkörper 26 infolge des Staudrucks in die zweite Position, so dass der Siebkörper 26 auf dem Ventilsitz 30 aufsitzt und Blutgerinnsel zurückgehalten werden. Wenn das Blut hingegen an dem zweiten Anschluss 23 in den Gehäusekörper 21 strömt, bewegt sich der Siebkörper 26 infolge des Staudrucks in die erste Position, so dass der Siebkörper von dem Blut umströmt wird. Dadurch wird ein Strömungsweg unter Umgehung des Siebkörpers geschaffen, so dass der Strömungswiderstand verringert ist.

Der erste Blutgerinnselfänger 15A ist in der ersten Blutleitung 5 derart angeordnet, dass der mit dem ersten Patientenanschluss 8 verbundene Abschnitt der ersten Blutleitung 5 an dem zweiten Anschluss 23 des Gehäusekörpers 21 angeschlossen ist und der mit dem Anschluss der Blutbehandlungseinheit 1 verbundene Abschnitt der ersten Blutleitung 5 an dem ersten Anschluss 22 des Gehäusekörpers 21 angeschlossen ist, so dass das Blut in der "normalen" Flussrichtung an dem Filtersieb 28 vorbei durch den ersten Blutgerinnselfänger 15A strömen kann, in der "umgekehrten" Flussrichtung aber Blutgerinnsel zurückgehalten werden.

Der zweite Blutgerinnselfänger 15B ist in der zweiten Blutleitung 7 derart angeordnet, dass der mit dem zweiten Patientenanschluss 9 verbundene Abschnitt der zweiten Blutleitung 7 an dem zweiten Anschluss 23 des Gehäusekörpers 21 angeschlossen ist und der mit dem Anschluss der Blutbehandlungseinheit 1 verbundene Abschnitt der zweiten Blutleitung 7 an dem ersten Anschluss 22 des Gehäusekörpers 21 angeschlossen ist, so dass in der "normalen" Flussrichtung Blutgerinnsel durch den zweiten Blutgerinnselfänger 15B zurückgehalten werden, in der "umgekehrten" Flussrichtung das Blut aber an dem Filtersieb 28 vorbei strömen kann.

Die Figuren 3A und 3B zeigen in vereinfachter schematischer Darstellung einen Schnitt durch ein zweites Ausführungsbeispiel des Blutgerinnselfängers, das sich von der ersten Ausführungsform durch die Ausbildung des Gehäusekörpers 21 und des Siebkörpers 26 unterscheidet. Die einander entsprechenden Teile sind mit denselben Bezugszeichen versehen. Bei dem zweiten Ausführungsbeispiel hat der vorzugsweise rotationssymmetrische Gehäusekörper 21 des Blutgerinnselfängers eine langgestreckte Form, wobei der Gehäusekörper auf der dem ersten Anschluss 22 zugewandte Seite kegelförmig und auf der dem zweiten Anschluss 23 zugewandte Seite eiförmig ausgebildet ist. Das Sieb 28 des Siebkörpers 26 ist bei dem zweiten Ausführungsbeispiel in der Form eines Fingerhuts ausgebildet, so dass die Siebfläche vergrößert ist.

Die Ausbildung des Gehäusekörpers 21 und des Siebkörpers 26 des Blutgerinnselfängers 15A, 15B ist nicht auf die oben beschriebenen Formen beschränkt. Gehäusekörper und Siebkörper können auch jede andere Form haben, solange in der entgegengesetzten Flussrichtung ein Strömungsweg unter Umgehung des Siebkörpers geschaffen wird.

## Patentansprüche

1. Vorrichtung zur extrakorporalen Blutbehandlung mit
einer Blutbehandlungseinheit (1), die mindestens ein Kompartiment (3) aufweist,
einer an einen Anschluss des Kompartiments (3) angeschlossenen ersten Blutleitung (5), die einen ersten Patientenanschluss (8) aufweist,
einer an einen Anschluss des Kompartiments (3) angeschlossenen zweiten Blutleitung (7), die einen zweiten Patientenanschluss (9) aufweist,
einer Blutpumpe zum Fördern von Blut,
einer Einrichtung (14A, 14B) zum Messen einer physikalischen oder chemischen Kenngröße,
einer mit der Blutpumpe (10) und der Einrichtung (14A, 14B) zum Messen einer physikalischen oder chemischen Kenngröße verbundenen Steuer- und Auswerteinheit (12), die derart konfiguriert ist, dass
die Blutpumpe (10) in einem ersten Betriebsmodus mit einem normalen Blutfluss betreibbar ist, so dass Blut von dem ersten Patientenanschluss (8) zu der Blutbehandlungseinheit (1) und von der Blutbehandlungseinheit zu dem zweiten Patientenanschluss (9) strömt,
die Blutpumpe (10) in einem zweiten Betriebsmodus mit einem umgekehrten Blutfluss betreibbar ist, so dass Blut von dem zweiten Patientenanschluss (9) zu der Blutbehandlungseinheit (1) und von der Blutbehandlungseinheit zu dem ersten Patientenanschluss (8) strömt, und
aus der gemessenen physikalischen oder chemischen Kenngröße bei normalem und umgekehrtem Blutfluss der hämodynamische Parameter bestimmt wird,
**dadurch gekennzeichnet, dass**
zumindest in der ersten Blutleitung (5) eine Vorrichtung (15A) zum Sammeln von Blutgerinnseln vorgesehen ist, wobei die Vorrichtung (15A) zum Sammeln von Blutgerinnseln einen Gehäusekörper (21) mit einem ersten Anschluss (22) und einem zweiten Anschluss (23) und einem im Strömungsweg des Bluts zwischen den beiden Anschlüssen angeordneten Siebkörper (26) zum Fangen der Blutgerinnsel aufweist, wobei die Vorrichtung zum Sammeln von Blutgerinnseln derart ausgebildet ist, dass sich in dem Gehäusekörper (21) ein den Siebkörper (26) umgehender Strömungsweg ausbildet, wenn Blut an dem zweiten Anschluss zufließt und an dem ersten Anschluss abfließt, und ein sich den Siebkörper umgehender Strömungsweg nicht ausbildet, wenn Blut an dem ersten Anschluss zufließt und an dem zweiten Anschluss abfließt.

2. Vorrichtung zur extrakorporalen Blutbehandlung nach Anspruch 1, **dadurch gekennzeichnet, dass** der Siebkörper (26) als Ventilkörper ausgebildet ist und in dem Gehäusekörper (21) ein Ventilsitz (30) für den Siebkörper ausgebildet ist, wobei der Siebkörper (26) in dem Gehäusekörper (21) derart bewegbar angeordnet ist, dass der Siebkörper auf dem Ventilsitz aufsitzt, wenn Blut an dem ersten Anschluss (22) zufließt und an dem zweiten Anschluss (23) abfließt, und von dem Ventilsitz abgehoben ist, wenn Blut an dem zweiten Anschluss (23) zufließt und an dem ersten Anschluss (22) abfließt, so dass sich in dem Gehäusekörper ein den Siebkörper umgehender Strömungsweg ausbildet.

3. Vorrichtung zur extrakorporalen Blutbehandlung nach Anspruch 2, **dadurch gekennzeichnet, dass** der Siebkörper (26) im Strömungsweg des Bluts zwischen dem ersten und zweiten Anschluss (22, 23) bewegbar angeordnet ist, wobei die Bewegung des Siebkörpers (26) in dem Gehäusekörper (21) auf einen Bereich zwischen einer ersten und einer zweiten Endposition beschränkt ist, wobei der Siebkörper (26) im Strömungsweg des Bluts zwischen dem ersten und zweiten Anschluss (22, 23) mit langgestreckten, flexiblen Fixierungselementen (29) an dem Gehäusekörper (21) fixiert ist, deren eine Enden mit dem Siebkörper und deren andere Enden mit dem Gehäusekörper verbunden sind, wobei die Fixierungselemente (29) um dessen Umfang angeordnet sind.

4. Vorrichtung zur extrakorporalen Blutbehandlung nach einem der Ansprüche 1 bis 3, **dadurch gekennzeichnet, dass** der mit dem ersten Patientenanschluss (8) verbundene Abschnitt der ersten Blutleitung (5) an dem zweiten Anschluss (23) des Gehäusekörpers (21) der Vorrichtung (15A) zum Sammeln von Blutgerinnseln angeschlossen ist und der mit dem Anschluss der Blutbehandlungseinheit (1) verbundene Abschnitt der ersten Blutleitung (5) an dem ersten Anschluss (22) des Gehäusekörpers (21) der ersten Vorrichtung (15A) zum Sammeln von Blutgerinnseln angeschlossen ist.

5. Vorrichtung zur extrakorporalen Blutbehandlung nach einem der Ansprüche 1 bis 4, **dadurch gekennzeichnet, dass** in der zweiten Blutleitung (5) eine Vorrichtung (15B) zum Sammeln von Blutgerinnseln vorgesehen ist, wobei der mit der Blutbehandlungseinheit (1) verbundene Abschnitt der zweiten Blutleitung (7) an dem ersten Anschluss (22) des Gehäusekörpers (21) der zweiten Vorrichtung (15B) zum Sammeln von Blutgerinnseln angeschlossen ist und der mit dem zweiten Patientenanschluss (9) verbundene Abschnitt der zweiten Blutleitung (7) an dem zweiten Anschluss (23) des Gehäusekörpers (21) der zweiten Vorrichtung (15B) zum Sammeln von Blutgerinnseln angeschlossen ist.

6. Vorrichtung zur extrakorporalen Blutbehandlung nach einem der Ansprüche 1 bis 5, **dadurch gekennzeichnet, dass** ein erster Drucksensor (13A) zum Messen des Drucks in der ersten Blutleitung (5) mit der Steuer- und Auswerteeinheit (12) verbunden ist, so dass die Steuer- und Auswerteeinheit (12) die Messwerte des ersten Drucksensors (13A) empfängt, und die Steuer- und Auswerteeinheit (12) derart konfiguriert ist, dass der mit dem ersten Drucksensor (13A) in dem zweiten Betriebsmodus gemessene Druck mit einem vorgegebenen Grenzwert verglichen wird, wobei ein Signal erzeugt wird, wenn der mit dem ersten Drucksensor gemessene Druck den vorgegebenen Grenzwert überschreitet.

7. Vorrichtung zur extrakorporalen Blutbehandlung nach einem der Ansprüche 1 bis 5, **dadurch gekennzeichnet, dass** ein erster Drucksensor (13A) zum Messen des Drucks in der ersten Blutleitung (5) und ein zweiter Drucksensor (13B) zum Messen des Drucks in der zweiten Blutleitung (7) mit der Steuer- und Auswerteeinheit (12) verbunden sind, so dass die Steuer- und Auswerteeinheit (12) die Messwerte des ersten und zweiten Drucksensors (13A, 13B) empfängt, und die Steuer- und Auswerteeinheit (12) derart konfiguriert ist, dass der mit dem ersten Drucksensor (13A) in dem zweiten Betriebsmodus gemessene Druck mit dem Druck verglichen wird, der mit dem zweiten Drucksensor (13B) in dem ersten Betriebsmodus gemessen wird, wobei ein Signal erzeugt wird, wenn der mit dem ersten Drucksensor (13A) gemessene Druck den mit dem zweiten Drucksensor (13B) gemessenen Druck um einen vorgegebenen Betrag oder Faktor überschreitet.

8. Vorrichtung zur extrakorporalen Blutbehandlung nach Anspruch 6 oder 7, **dadurch gekennzeichnet, dass** die Steuer- und Auswerteeinheit (12) derart konfiguriert ist, dass die Blutpumpe (10) von dem umgekehrten Blutfluss auf den normalen Blutfluss umgeschaltet wird, wenn das Signal erzeugt wird.

9. Vorrichtung zum Sammeln von Blutgerinnseln für eine Blutleitung zum Zuführen oder Abführen von Blut in eine Blutbehandlungseinheit einer extrakorporalen Blutbehandlungsvorrichtung, **dadurch gekennzeichnet, dass** die Vorrichtung (15A, 15B) einen Gehäusekörper (21) mit einem ersten Anschluss (22) und einem zweiten Anschluss (23) und einem im Strömungsweg des Bluts zwischen den beiden Anschlüssen angeordneten Siebkörper (26) zum Fangen der Blutgerinnsel aufweist, wobei die Vorrichtung (15A, 15B) zum Sammeln von Blutgerinnseln derart ausgebildet ist, dass sich in dem Gehäusekörper (21) ein den Siebkörper (26) umgehender Strömungsweg ausbildet, wenn Blut an dem zweiten Anschluss (23) zufließt und an dem ersten Anschluss (22) abfließt, und ein sich den Siebkörper (26) umgehender Strömungsweg nicht ausbildet, wenn Blut an dem ersten Anschluss (22) zufließt und an dem zweiten Anschluss (23) abfließt.

10. Vorrichtung nach Anspruch 9, **dadurch gekennzeichnet, dass** der Siebkörper (26) als Ventilkörper ausgebildet ist und in dem Gehäusekörper (21) ein Ventilsitz (30) für den Siebkörper ausgebildet ist, wobei der Siebkörper (26) in dem Gehäusekörper (21) derart bewegbar angeordnet ist, dass der Siebkörper (26) auf dem Ventilsitz (30) aufsitzt, wenn Blut an dem ersten Anschluss (22) zufließt und an dem zweiten Anschluss (23) abfließt, und von dem Ventilsitz (30) abgehoben ist, wenn Blut an dem zweiten Anschluss (23) zufließt und an dem ersten Anschluss (22) abfließt, so dass sich in dem Gehäusekörper ein den Siebkörper umgehender Strömungsweg ausbildet.

11. Vorrichtung nach Anspruch 10, **dadurch gekennzeichnet, dass** der Siebkörper im Strömungsweg des Bluts zwischen dem ersten und zweiten Anschluss (22, 23) bewegbar angeordnet ist, wobei die Bewegung des Siebkörpers (26) in dem Gehäusekörper (21) auf einen Bereich zwischen einer ersten und einer zweiten Endposition beschränkt ist.

12. Vorrichtung nach Anspruch 11, **dadurch gekennzeichnet, dass** der Siebkörper im Strömungsweg des Bluts zwischen dem ersten und zweiten Anschluss (22, 23) mit langgestreckten, flexiblen Fixierungselementen (29) an dem Gehäusekörper (21) fixiert ist, deren eine Enden mit dem Siebkörper (26) und deren andere Enden mit dem Gehäusekörper (21) verbunden sind, wobei die Fixierungselemente (29) um dessen Umfang angeordnet sind.

13. Vorrichtung nach Anspruch 12, **dadurch gekennzeichnet, dass** der Gehäusekörper (21) einen kreisförmigen Querschnitt aufweist und der Siebkörper (26) ein Halteteil (27) mit einem kreisförmigen Querschnitt aufweist, in das ein Sieb (28) eingesetzt ist.

14. Vorrichtung nach Anspruch 13, **dadurch gekennzeichnet, dass** das Halteteil mit dem Sieb (28) als flacher Körper oder in der Form eines Fingerhuts ausgebildet ist.

15. Vorrichtung zur extrakorporalen Blutbehandlung nach einem der Ansprüche 1 bis 8, **dadurch gekennzeichnet, dass** der hämodynamische Parameter der Shuntfluss ist.

## Claims

1. Apparatus for extracorporeal blood treatment, comprising
a blood treatment unit (1) that comprises at least one compartment (3),
a first blood line (5) that is connected to a connection of the compartment (3) and comprises a first patient connection (8),
a second blood line (7) that is connected to a connection of the compartment (3) and comprises a second patient connection (9),
a blood pump for conveying blood,
a device (14A, 14B) for measuring a physical or chemical characteristic variable,
a control and evaluation unit (12) that is connected to the blood pump (10) and to the device (14A, 14B) for measuring a physical or chemical characteristic variable, and is designed such that
the blood pump (10) can be operated in a first operating mode having a normal blood flow, such that blood flows from the first patient connection (8) to the blood treatment unit (1) and from the blood treatment unit to the second patient connection (9),
the blood pump (10) can be operated in a second operating mode having a reversed blood flow, such that blood flows from the second patient connection (9) to the blood treatment unit (1) and from the blood treatment unit to the first patient connection (8), and
the haemodynamic parameter is determined from the measured physical or chemical characteristic variable during normal and reversed blood flow,
**characterised in that**
an apparatus (15A) for collecting blood clots is provided at least in the first blood line (5), the apparatus (15A) for collecting blood clots comprising a housing body (21) having a first connection (22) and a second connection (23), and a screen body (26) for catching the blood clots that is arranged between the two connections in the flow path of the blood, the apparatus for collecting blood clots being designed such that a flow path bypassing the screen body (26) forms in the housing body (21) when blood flows in at the second connection and out at the first connection, and a flow path bypassing the screen body does not form when blood flows in at the first connection and out at the second connection.

2. Apparatus for extracorporeal blood treatment according to claim 1, **characterised in that** the screen body (26) is designed as a valve body and a valve seat (30) for the screen body is formed in the housing body (21), the screen body (26) being movably arranged in the housing body (21) such that the screen body rests on the valve seat when blood flows in at the first connection (22) and out at the second connection (23), and is lifted off the valve seat when blood flows in at the second connection (23) and out at the first connection (22), such that a flow path bypassing the screen body forms in the housing body.

3. Apparatus for extracorporeal blood treatment according to claim 2, **characterised in that** the screen body (26) is movably arranged between the first and second connection (22, 23) in the flow path of the blood, the movement of the screen body (26) in the housing body (21) being restricted to a range between a first and a second end position, the screen body (26) being fixed to the housing body (21) between the first and second connection (22, 23) in the flow path of the blood by means of elongate, flexible fixing elements (29), one end of each of which elements being connected to the screen body and the other end of each of which elements being connected to the housing body, the fixing elements (29) being arranged around the periphery of said screen body.

4. Apparatus for extracorporeal blood treatment according to any of claims 1 to 3, **characterised in that** the portion of the first blood line (5) connected to the first patient connection (8) is connected to the second connection (23) of the housing body (21) of the apparatus (15A) for collecting blood clots, and the portion of the first blood line (5) connected to the connection of the blood treatment unit (1) is connected to the first connection (22) of the housing body (21) of the first apparatus (15A) for collecting blood clots.

5. Apparatus for extracorporeal blood treatment according to any of claims 1 to 4, **characterised in that** an apparatus (15B) for collecting blood clots is provided in the second blood line (5), the portion of the second blood line (7) connected to the blood treatment unit (1) being connected to the first connection (22) of the housing body (21) of the second apparatus (15B) for collecting blood clots, and the portion of the second blood line (7) connected to the second patient connection (9) being connected to the second connection (23) of the housing body (21) of the second apparatus (15B) for collecting blood clots.

6. Apparatus for extracorporeal blood treatment according to any of claims 1 to 5, **characterised in that** a first pressure sensor (13A) for measuring the pressure in the first blood line (5) is connected to the control and evaluation unit (12) such that the control and evaluation unit (12) receives the measured values from the first pressure sensor (13A), and the control and evaluation unit (12) is designed such that the pressure measured by the first pressure sensor (13A) in the second operating mode is compared with a predetermined threshold value, a signal being generated when the pressure measured by the first pressure sensor exceeds the predetermined threshold value.

7. Apparatus for extracorporeal blood treatment according to any of claims 1 to 5, **characterised in that** a first pressure sensor (13A) for measuring the pressure in the first blood line (5) and a second pressure sensor (13B) for measuring the pressure in the second blood line (7) are connected to the control and evaluation unit (12) such that the control and evaluation unit (12) receives the measured values from the first and second pressure sensor (13A, 13B), and the control and evaluation unit (12) is designed such that the pressure measured by the first pressure sensor (13A) in the second operating mode is compared with the pressure measured by the second pressure sensor (13B) in the first operating mode, a signal being generated when the pressure measured by the first pressure sensor (13A) exceeds the pressure measured by the second pressure sensor (13B) by a predetermined amount or factor.

8. Apparatus for extracorporeal blood treatment according to either claim 6 or claim 7, **characterised in that** the control and evaluation unit (12) is designed such that the blood pump (10) is switched from the reversed blood flow to the normal blood flow when the signal is generated.

9. Apparatus for collecting blood clots in a blood line for supplying or removing blood in a blood treatment unit of an extracorporeal blood treatment apparatus, **characterised in that** the apparatus (15A, 15B) comprises a housing body (21) having a first connection (22) and a second connection (23), and a screen body (26) for catching the blood clots that is arranged between the two connections in the flow path of the blood, the apparatus (15A, 15B) for collecting blood clots being designed such that a flow path bypassing the screen body (26) forms in the housing body (21) when blood flows in at the second connection (23) and out at the first connection (22), and a flow path bypassing the screen body (26) does not form when blood flows in at the first connection (22) and out at the second connection (23).

10. Apparatus according to claim 9, **characterised in that** the screen body (26) is designed as a valve body and a valve seat (30) for the screen body is formed in the housing body (21), the screen body (26) being movably arranged in the housing body (21) such that the screen body (26) rests on the valve seat (30) when blood flows in at the first connection (22) and out at the second connection (23), and is lifted off the valve seat (30) when blood flows in at the second connection (23) and out at the first connection (22), such that a flow path bypassing the screen body forms in the housing body.

11. Apparatus according to claim 10, **characterised in that** the screen body is movably arranged between the first and second connection (22, 23) in the flow path of the blood, the movement of the screen body (26) in the housing body (21) being restricted to a range between a first and a second end position.

12. Apparatus according to claim 11, **characterised in that** the screen body is fixed to the housing body (21) between the first and second connection (22, 23) in the flow path of the blood by means of elongate, flexible fixing elements (29), one end of each of which elements is connected to the screen body (26) and the other end of each of which elements is connected to the housing body (21), the fixing elements (29) being arranged around the periphery of said screen body.

13. Apparatus according to claim 12, **characterised in that** the housing body (21) has a circular cross section and the screen body (26) comprises a retaining part (27) having a circular cross section, in which part a screen (28) is inserted.

14. Apparatus for extracorporeal blood treatment according to claim 8, **characterised in that** the retaining part is designed having the screen (28) as a planar body or in the shape of a thimble.

15. Apparatus according to any of claims 1 to 8, **characterised in that** the haemodynamic parameter is the shunt flow.

## Revendications

1. Dispositif pour le traitement extracorporel du sang avec
une unité de traitement du sang (1) qui présente au moins un compartiment (3), une première conduite de sang (5) raccordée à un raccordement du compartiment (3) qui présente un premier raccord au patient (8),
une deuxième conduite de sang (7) raccordée à un raccordement du compartiment (3) qui présente un deuxième raccord au patient (9),
une pompe à sang pour transporter du sang,
un équipement (14A, 14B) pour mesurer une grandeur caractéristique physique ou chimique,
une unité de commande et d'évaluation (12) reliée à la pompe à sang (10) et à l'équipement (14A, 14B) pour mesurer une grandeur caractéristique physique ou chimique qui est configurée de telle sorte que
la pompe à sang (10) dans un premier mode de fonctionnement peut fonctionner avec un débit sanguin normal, de sorte que du sang circule à partir du premier raccord au patient (8) vers l'unité de traitement du sang (1) et à partir de l'unité de traitement du sang vers le deuxième raccord au patient (9),
la pompe à sang (10) dans un deuxième mode de fonctionnement peut fonctionner avec un débit sanguin inverse, de sorte que du sang circule à partir du deuxième raccord au patient (9) vers l'unité de traitement du sang (1) et à partir de l'unité de traitement du sang vers le premier raccord au patient (8), et
le paramètre hémodynamique est déterminé à partir de la grandeur caractéristique physique ou chimique mesurée pour un débit sanguin normal et inverse,
**caractérisé en ce que**
un dispositif (15A) pour collecter des caillots de sang est prévu au moins dans la première conduite de sang (5), le dispositif (15A) pour collecter des caillots de sang présentant un corps de boîtier (21) avec un premier raccord (22) et un deuxième raccord (23) et un corps de tamisage (26) disposé dans la voie d'écoulement du sang entre les deux raccords pour capturer les caillots de sang, dans lequel le dispositif pour collecter des caillots de sang est réalisé de telle sorte qu'une voie d'écoulement contournant le corps de tamisage (26) se forme dans le corps de boîtier (21) lorsque du sang afflue via le deuxième raccord et s'écoule pour ressortir par le premier raccord, et une voie d'écoulement contournant le corps de tamisage ne se forme pas lorsque du sang afflue via le premier raccord et s'écoule pour ressortir par le deuxième raccord.

2. Dispositif pour le traitement extracorporel du sang selon la revendication 1, **caractérisé en ce que** le corps de tamisage (26) est réalisé en tant que corps de valve et un siège de valve (30) pour le corps de tamisage est réalisé dans le corps de boîtier (21), dans lequel le corps de tamisage (26) est disposé mobile dans le corps de boîtier (21), de telle sorte que le corps de tamisage repose sur le siège de valve lorsque du sang afflue via le premier raccord (22) et s'écoule pour ressortir par le deuxième raccord (23), et est soulevé du siège de valve lorsque du sang afflue via le deuxième raccord (23) et s'écoule pour ressortir par le premier raccord (22), de sorte qu'une voie d'écoulement contournant le corps de tamisage se forme dans le corps de boîtier.

3. Dispositif pour le traitement extracorporel du sang selon la revendication 2, **caractérisé en ce que** le corps de tamisage (26) est disposé mobile dans la voie d'écoulement du sang entre le premier et deuxième raccord (22, 23), le mouvement du corps de tamisage (26) dans le corps de boîtier (21) étant limité à une zone entre une première et une deuxième position d'extrémité, dans lequel le corps de tamisage (26) est fixé sur le corps de boîtier (21) dans la voie d'écoulement du sang entre le premier et deuxième raccord (22, 23) au moyen d'éléments de fixation (29) allongés, flexibles, dont une extrémité est reliée au corps de tamisage et l'autre extrémité au corps de boîtier, ce grâce à quoi les éléments de fixation (29) sont disposés autour de la périphérie de ce corps de tamisage.

4. Dispositif pour le traitement extracorporel du sang selon l'une quelconque des revendications 1 à 3, **caractérisé en ce que** la partie de la première conduite de sang (5) reliée au premier raccord au patient (8) est raccordée au deuxième raccord (23) du corps de boîtier (21) du dispositif (15A) pour collecter des caillots de sang et la partie de la première conduite de sang (5) reliée au raccord de l'unité de traitement du sang (1) est raccordée au premier raccord (22) du corps de boîtier (21) du premier dispositif (15A) pour collecter des caillots de sang.

5. Dispositif pour le traitement extracorporel du sang selon l'une quelconque des revendications 1 à 4, **caractérisé en ce qu'**un dispositif (15B) pour collecter des caillots de sang est prévu dans la deuxième conduite de sang (5), dans lequel la partie de la deuxième conduite de sang (7) reliée à l'unité de traitement du sang (1) est raccordée au premier raccord (22) du corps de boîtier (21) du deuxième dispositif (15B) pour collecter des caillots de sang et la partie de la deuxième conduite de sang (7) reliée au deuxième raccord au patient (9) est raccordée au deuxième raccord (23) du corps de boîtier (21) du deuxième dispositif (15B) pour collecter des caillots de sang.

6. Dispositif pour le traitement extracorporel du sang selon l'une quelconque des revendications 1 à 5, **caractérisé en ce qu'**un premier capteur de pression (13A) pour mesurer la pression dans la première conduite de sang (5) est relié à l'unité de commande et d'évaluation (12), de sorte que l'unité de commande et d'évaluation (12) reçoit les valeurs de mesure du premier capteur de pression (13A), et l'unité de commande et d'évaluation (12) est configurée de telle sorte que la pression mesurée avec le premier capteur de pression (13A) dans le deuxième mode de fonctionnement est comparée à une valeur limite prédéfinie, un signal étant généré lorsque la pression mesurée avec le premier capteur de pression dépasse la valeur limite prédéfinie.

7. Dispositif pour le traitement extracorporel du sang selon l'une quelconque des revendications 1 à 5, **caractérisé en ce qu'**un premier capteur de pression (13A) pour mesurer la pression dans la première conduite de sang (5) et un deuxième capteur de pression (13B) pour mesurer la pression dans la deuxième conduite de sang (7) sont reliés à l'unité de commande et d'évaluation (12), de sorte que l'unité de commande et d'évaluation (12) reçoit les valeurs de mesure du premier et deuxième capteur de pression (13A, 13B), et l'unité de commande et d'évaluation (12) est configurée de telle sorte que la pression mesurée avec le premier capteur de pression (13A) dans le deuxième mode de fonctionnement est comparée à la pression qui est mesurée avec le deuxième capteur de pression (13B) dans le premier mode de fonctionnement, un signal étant généré lorsque la pression mesurée avec le premier capteur de pression (13A) dépasse la pression mesurée avec le deuxième capteur de pression (13B) d'un montant ou facteur prédéfini.

8. Dispositif pour le traitement extracorporel du sang selon la revendication 6 ou 7, **caractérisé en ce que** l'unité de commande et d'évaluation (12) est configurée de telle sorte que la pompe à sang (10) est basculée du débit sanguin inverse au le débit sanguin normal lorsque le signal est généré.

9. Dispositif pour collecter des caillots de sang pour une conduite de sang afin d'amener ou évacuer du sang dans une unité de traitement du sang d'un dispositif pour le traitement extracorporel du sang, **caractérisé en ce que** le dispositif (15A, 15B) présente un corps de boîtier (21) avec un premier raccord (22) et un deuxième raccord (23) et un corps de tamisage (26) disposé dans la voie d'écoulement du sang entre les deux raccords pour capturer les caillots de sang, dans lequel le dispositif (15A, 15B) pour collecter des caillots de sang est réalisé de telle sorte qu'une voie d'écoulement contournant le corps de tamisage (26) se forme dans le corps de boîtier (21) lorsque du sang afflue via le deuxième raccord (23) et s'écoule pour ressortir par le premier raccord (22), et une voie d'écoulement contournant le corps de tamisage (26) ne se forme pas lorsque du sang afflue via le premier raccord (22) et s'écoule pour ressortir par le deuxième raccord (23).

10. Dispositif selon la revendication 9, **caractérisé en ce que** le corps de tamisage (26) est réalisé en tant que corps de valve et un siège de valve (30) pour le corps de tamisage est réalisé dans le corps de boîtier (21), le corps de tamisage (26) étant disposé mobile dans le corps de boîtier (21), de telle sorte que le corps de tamisage (26) repose sur le siège de valve (30) lorsque du sang afflue via le premier raccord (22) et s'écoule pour ressortir par le deuxième raccord (23), et est soulevé du siège de valve (30) lorsque du sang afflue via le deuxième raccord (23) et s'écoule pour ressortir par le premier raccord (22), de sorte qu'une voie d'écoulement contournant le corps de tamisage se forme dans le corps de boîtier.

11. Dispositif selon la revendication 10, **caractérisé en ce que** le corps de tamisage est disposé mobile dans la voie d'écoulement du sang entre le premier et deuxième raccord (22, 23), dans lequel le mouvement du corps de tamisage (26) dans le corps de boîtier (21) est limité à une zone entre une première et une deuxième position d'extrémité.

12. Dispositif selon la revendication 11, **caractérisé en ce que** le corps de tamisage (26) est fixé sur le corps de boîtier (21) dans la voie d'écoulement du sang entre le premier et deuxième raccord (22, 23) au moyen d'éléments de fixation (29) allongés, flexibles, dont une extrémité est reliée au corps de tamisage et l'autre extrémité au corps de boîtier (21), ce grâce à quoi les éléments de fixation (29) sont disposés autour de la périphérie de ce corps de tamisage.

13. Dispositif selon la revendication 12, **caractérisé en ce que** le corps de boîtier (21) présente une section transversale circulaire et le corps de tamisage (26) présente une pièce de retenue (27) avec une section transversale circulaire, dans laquelle est inséré un tamis (28).

14. Dispositif selon la revendication 13, **caractérisé en ce que** la pièce de retenue avec le tamis (28) est réalisée en tant que corps plat ou sous la forme d'un dé à coudre.

15. Dispositif pour le traitement extracorporel du sang selon l'une quelconque des revendications 1 à 8, **caractérisé en ce que** le paramètre hémodynamique est le flux de shunt.
